# Europäisches Patentamt

(19) **European Patent Office**

**Office européen des brevets**

(11) Veröffentlichungsnummer : **0 339 320 B1**

(12)

# EUROPÄISCHE PATENTSCHRIFT

(45) Veröffentlichungstag der Patentschrift :
03.07.91 Patentblatt 91/27

(51) Int. Cl.⁵ : **A61F 2/30**

(21) Anmeldenummer : **89105993.3**

(22) Anmeldetag : **05.04.89**

(54) **Prothesenschaft.**

(30) Priorität : **27.04.88 DE 8805583 U**

(43) Veröffentlichungstag der Anmeldung :
**02.11.89 Patentblatt 89/44**

(45) Bekanntmachung des Hinweises auf die
Patenterteilung :
**03.07.91 Patentblatt 91/27**

(84) Benannte Vertragsstaaten :
**AT CH DE ES FR GB IT LI NL**

(56) Entgegenhaltungen :
**EP-A- 0 044 915
WO-A-86/00011
DE-A- 3 445 738
DE-B- 2 461 339**

(73) Patentinhaber : **THERA Patent GmbH & Co. KG
Gesellschaft für industrielle Schutzrechte
Griesberg 2
W-8031 Seefeld 1 (DE)**

(72) Erfinder : **Wilhelm, Klaus
Pettenkoferstrasse 8a
W-8000 München 2 (DE)**
Erfinder : **Bauer, Johann
Pettenkoferstrasse 8a
W-8000 München 2 (DE)**
Erfinder : **Schmitt, Werner
Prinzenweg 10
W-8031 Starnberg (DE)**
Erfinder : **Herold, Wolf-Dietrich
Höhenweg 13
W-8031 Seefeld 2 (DE)**
Erfinder : **Koran, Peter
Tassiloring 7
W-8120 Weilheim (DE)**
Erfinder : **Wanek, Erich
An der Breite 9
W-8031 Seefeld (DE)**
Erfinder : **Gasser, Oswald
Höhenstrasse 10
W-8031 Seefeld (DE)**

(74) Vertreter : **Strehl, Schübel-Hopf, Groening
Maximilianstrasse 54 Postfach 22 14 55
W-8000 München 22 (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft einen Prothesenschaft zur Implantation einer Prothese, insbesondere eines künstlichen Hüft-, Knie- oder Fingergelenkteils mit Hilfe eines Knochenzements.

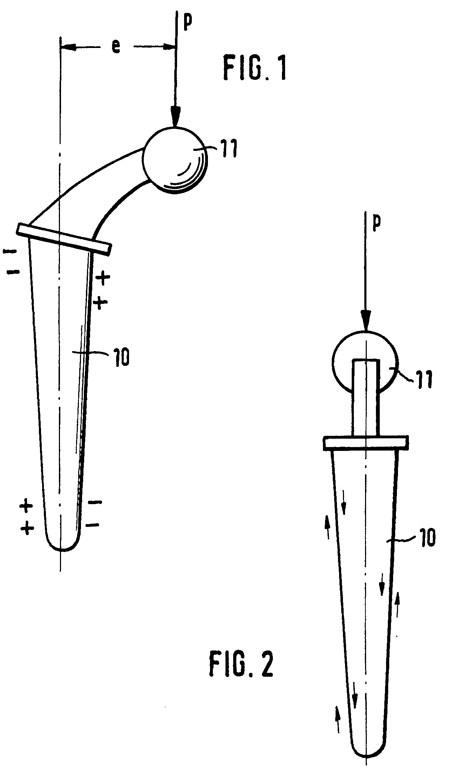

Bei herkömmlichen Prothesen weist der im Knochen zu verankernde Schaft eine im wesentlichen glatte, konisch gestaltete Oberfläche auf. In Figur 1 und 2 ist am Beispiel einer Hüftprothese schematisch dargestellt, welche Haltekräfte an der Oberfläche des Schaftes 10 bei Beaufschlagung der Gelenkkugel 11 mit einer Kraft P auftreten. Diese Haltekräfte lassen sich in die aus dem Biegemoment, d.h. der Kraft P mal dem Abstand e der Kraftwirkungsrichtung von der Schaftachse, resultierende Druckkräfte ( + ) und Zugkräfte ( – ) und die aus der Axialkraft P resultierenden Scherkräfte (↑↓) an der Schaftoberfläche zerlegen. Die tatsächliche Beanspruchung des den Schaft verankernden Knochenzements ergibt sich aus der Überlagerung dieser Druck-, Zug- und Scherkräfte. Ein Prothesenschaft gemäß dem Oberbegriff des Anspruchs 1 ist aus der WO-A-8 600 011 bekannt.

Aus EP-A-0 212 084 ist es bekannt, zur Verankerung eines insgesamt konischen Prothesenschaftes mit Hilfe von Knochenzement die Schaftoberfläche zusätzlich mit zylindrischen Vertiefungen zu versehen.

Bekannte Knochenzemente können Druck- und Zugkräfte annähernd gleich gut übertragen und auch Scherkräfte verhältnismäßig problemlos aufnehmen. Ein gewichtiger Nachteil der bekannten Knochenzemente besteht aber darin, daß sie den Knochen angreifen, so daß die Lebensdauer der Verankerung begrenzt ist und eine spätere erneute Verankerung wegen des dann teilweise zerstörten Knochenendes nicht mehr ohne weiteres möglich ist.

Aus diesem Grund wird häufig versucht, Prothesen zement frei in den Knochen zum verankern. Aus DE-A-2 461 339 ist eine derartige Prothese bekannt, deren im wesentlichen aus einer flachen Metallplatte bestehender Schaft im Knochen an seinen Schmalseiten mit verrundeten Stufen versehen ist. Diese Stufen sind so ausgebildet, daß sie jeweils eine senkrecht zu der trajektoriell orientierten Spongiosastruktur gerichtete Kantenfläche zur unmittelbaren Übertragung der lokalen Druck- und Zugkräfte aufweist. Die so erzeugte, insgesamt etwa sägezahnartige Gestaltung der Kantenflächen soll auch bewirken, daß die Gesamtauflagefläche zwischen Schaft und Knochengewebe vergrößert und die daran wirkenden Drücke entsprechend kleiner werden. Die mit einem solchen Schaft angestrebte zementfreie Verankerung setzt aber ein gutes Wachstum des Knochengewebes voraus, damit die sägezahnartigen Schaftflächen in der gewünschten Weise sicher eingebettet werden. Selbst wenn diese Voraussetzung zunächst gegeben ist, besteht bei einer zementfreien Verankerung immer die Gefahr, daß sie sich im Lauf der Zeit lockert.

Aus DE-A-3 445 738 ist ferner ein generell hohlzylindrischer Knochendübel bekannt, der ein Innengewinde zum Einschrauben einer Knochenschraube aufweist und dessen Außenfläche von mehreren einander teilweise durchdringenden großen Kugelflächen gebildet ist und mit Axial- und/oder Querschlitzen sowie zusätzlichen Unebenheiten, insbesondere in Form kleiner Kugeln, versehen ist. Durch diese Gestaltung soll eine möglichst innige Verbindung mit dem verbliebenen Knochengewebe erreicht werden, damit der zementfrei verankerte Prothesenteil gut einwächst. Wiederum wird also ausreichendes Knochenwachstum vorausgesetzt und die dennoch immer vorhandene Gefahr des späteren Lockerns in Kauf genommen.

Neuerdings ist daran gedacht worden, als Knochenzemente Glasionomere einzusetzen, die bioinert sind und daher im Gegensatz zu den herkömmlichen Zementen das Knochengewebe nicht angreifen. Glasionomer-Knochenzemente weisen zwar sehr gute Druckfestigkeit, aber nur geringe Zugfestigkeit sowie ein ausgeprägtes Sprödbruchverhalten auf.

Der Erfindung liegt die Aufgabe zugrunde, einen Prothesenschaft derart zu gestalten, daß er sich unter Verwendung eines Glasionomer-Knochenzements sicher und dauerhaft im Knochen verankern läßt.

Die erfindungsgemäße Lösung dieser Aufgabe ist im Anspruch 1 gekennzeichnet. Danach ist die Schaftoberfläche so gestaltet, daß an ihr im wesentlichen nur Druckspannungen auftreten, während Zugspannungen im wesentlichen vermieden werden. Gleichzeitig werden Kanten und Ecken vermieden, die zu Kerbspannungen und damit zu Sprödbrüchen des Knochenzements führen könnten.

Vorteilhafte Ausgestaltungen der Erfindung im hinsichtlich einer möglichst sicheren formschlüssigen Verankerung sowie zur Vermeidung von Bereichen, an denen Zug- oder Kerbspannungen auftreten können, sind in den Unteransprüchen gekennzeichnet.

Ausführungsbeispiele der Erfindung werden nachstehend anhand der übrigen Zeichnungen näher erläutert, in denen

Figur 3 eine Hüftgelenksprothese,

Figur 4 eine Fingergelenksprothese,

Figur 5 eine der Figur 3 ähnliche, schematisierte Darstellung, und

Figur 6 einen Schnitt in der Ebene A-A der Figur 5 zeigt.

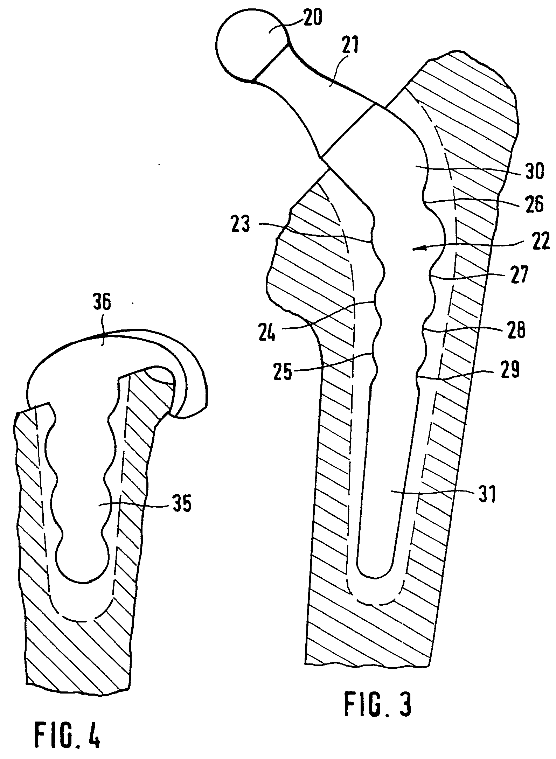

Bei der in Figur 3 dargestellten metallischen Hüftgelenksprothese ist die Gelenkkugel 20 über einen Schenkel 21 mit einem generell mit 22 bezeichneten Schaft verbunden. Gelenkkugel 20, Schenkel 21 und Schaft 22 können einstückig geformt oder auch aus einzelnen Teilen zusammengesetzt, z.B. zusammengeschraubt, sein.

Die Schaftoberfläche ist in ihrem oberen Teil aus mehreren ineinander übergehenden Kugelflächen 23, 24, 25 aufgebaut. Die Übergangsbereiche 26, 27, 28, 29 zwischen den einzelnen Kugelflächen 23...25, zwischen der obersten Kugelfläche 23 und dem mit dem Schenkel 21 verbundenen proximalen Schaftabschnitt 30 sowie zwischen der untersten Kugelfläche 25 und dem insgesamt leicht konisch geformten distalen Schaftabschnitt 31 sind als konkav verrundete Ringflächen gestaltet.

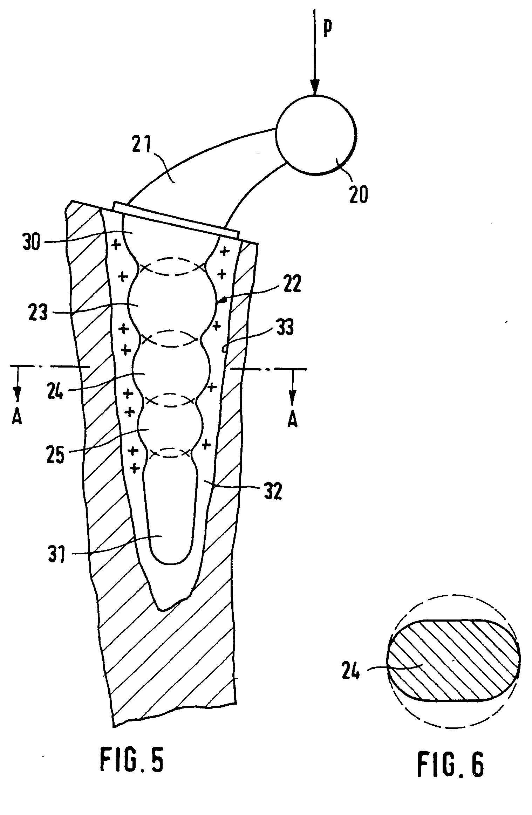

Die Formgebung des Schafts beruht auf der Erkenntnis, daß beim Eindrücken einer Kugel in ein viskoses Material ausschließlich radiale Druckkräfte entstehen. Dies gilt auch dann, wenn mehrere derartiger Kugeln längs der Schaftachse hintereinander angeordnet werden. Wie in der schematischen Darstellung der Figur 5 durch die Symbole ( + ) angedeutet, treten auch bei einer derartigen Anordnung mehrerer Kugeln an praktisch sämtlichen Stellen in dem mit 32 bezeichneten Knochenzement nur Druckkräfte auf. Beim Angreifen der in Figur 5 gezeigten Kraft P an der Gelenkkugel 20 erzeugt jede Kugel des Schafts mit ihrer unteren Hälfte ausschließlich Druckkräfte. Ferner stützt sich die jeweils obere Kugel über den Knochenzement 32 auf der unteren Kugel ab, so daß auch im Bereich zwischen den Kugeln Druckspannungen vorliegen.

Der schematischen Darstellung der Figur 5 ist ferner deutlich zu entnehmen, daß die Kugeln bzw. Kugelflächen 23... 25 vom proximalen zum distalen Schaftende hin abnehmenden Radius aufweisen und einander nur so weit durchdringen, daß die Mittelpunkte jeweils benachbarter Kugeln außerhalb des gegenseitigen Durchdringungsbereiches liegen. Ferner ist es zweckmäßig, daß die Kugeln – soweit dies der im Knochen verfügbare Hohlraum 33 zuläßt – jeweils möglichst große Radien aufweisen. Bei dem genannten Hohlraum 33 handelt es sich im wesentlichen um den vom Knochenmark befreiten natürlichen Hohlraum, dessen Fläche zur Erzielung einer innigeren Verbindung mit dem Knochenzement 32 angerauht sein kann.

Um zu verhindern, daß die Prothese um die Schaftachse relativ zum Knochen rotiert, ist mindestens eine der Kugeln in ihrem in Figur 6 gezeigten Querschnitt ellipsoidartig abgeflacht oder in sonstiger Weise asymmetrisch geformt. Damit die aus den Torsionsmomenten eingeleiteten Kräfte sicher aufgenommen werden können, ist dieser abgeflachte Körper in der Mitte des Längsschaftes angeordnet. In diesem Bereich können zusätzliche Kräfte am leichtesten aufgenommen werden, ohne daß ein Ausbrechen des Materials zu befürchten ist. Wie die Schnittlinie A-A zeigt, handelt es sich bei dem Ausführungsbeispiel nach Figur 5 bei dem abgeflachten Körper um die mittlere Kugel 24.

Figur 4 zeigt ein Beispiel für eine Gestaltung des Prothesenschafts 35 für ein Fingergelenk. Der Schaft 35 selbst ist – in entsprechender Verkleinerung – im wesentlichen analog dem Schaft 22 der Hüftgelenksprothese nach Figur 3 ausgebildet, wobei wegen der zur Verfügung stehenden geringeren Knochenlänge und wegen der geringeren auftretenden Kräfte der in Figur 3 gezeigte distale Schaftabschnitt 31 weggelassen ist. Der an den Schaft ansetzende proximale Gelenkteil 36 ist entsprechend der natürlichen Gelenkschale gestaltet.

## Ansprüche

1. Prothesenschaft in Gestalt eines massiven Formteils, dessen Oberfläche senkrecht zur Druckkraftrichtung verlaufende, über konkav verrundete Übergangsbereiche (26...29) ineinander übergehende Flächenbereiche aufweist, dadurch gekennzeichnet, daß die Schaftoberfläche von den Flächen mehrerer einander teilweise durchdringender Kugeln (23...25) gebildet ist, die mit ihren Mittelpunkten längs der Schaft-Mittellinie angeordnet sind, wobei die konkav verrundeten Übergangsbereiche (26...29) zwischen jeweils benachbarten Kugelflächen ausgebildet sind.

2. Prothesenschaft nach Anspruch 1, dadurch gekennzeichnet, daß die Mittelpunkte jeweils benachbarter Kugeln (23...25) außerhalb des jeweiligen Durchdringungsbereichs liegen.

3. Prothesenschaft nach Anspruch 1 oder 2, dadurch gekennzeichnet, daß die Kugeln (23...25) vom proximalen zum distalen Schaftende hin abnehmenden Radius haben.

4. Prothesenschaft nach einem der Ansprüche 1 bis 3, dadurch gekennzeichnet, daß die Kugeln (23...25) innerhalb des im Knochen verfügbaren Hohlraums (33) mit möglichst großem Radius ausgebildet sind.

5. Prothesenschaft nach einem der Ansprüche 1 bis 4, dadurch gekennzeichnet, daß im mittleren Teil der Schaftlänge ein Bereich (24) mit nicht-kreisförmigen Querschnitt vorgesehen ist.

## Claims

1. A prosthesis shaft shaped as a massive moulded part, the outer surface of which includes surface portions extending perpendicular to the direction of the pressure force and verging into each other through concavely rounded transitional zones (26...29), characterised in that the shaft surface is formed by the surfaces of a plurality of partially intersecting spheres (23...25) which have their centers arranged along the center line of the shaft, said concavely rounded transitional zones (26...29) being formed between respective adjacent spherical surfaces.

2. The prosthesis shaft of claim 1, characterised in that the centers of respective adjacent spheres (23...25) are disposed outside the respective region of intersection.

3. The prosthesis shaft of claim 1 or 2, characterised in that the spheres (23...25) have radii decreasing from the proximal to the distal end of the shaft.

4. The prosthesis shaft of any of claims 1 to 3, characterised in that the spheres (23...25) are formed with the largest radii possible within the cavity (33) available in the bone.

5. The prosthesis shaft of any of claims 1 to 4, characterised in that a region (24) of non-circular cross-section is provided in the middle part of the shaft length.

**Revendications**

1. Tige de prothèse sous forme d'une pièce massive dont la surface présente des zones de surfaces s'étendant perpendiculairement à la direction des forces de compression et se raccordant les unes aux autres par l'intermédiaire de zones (26...29) de transition arrondies concaves, caractérisée en ce que la surface de la tige est formée des surfaces de plusieurs boules (23...25) pénétrant partiellement l'une dans l'autre, et qui sont disposées avec leurs centres le long de la ligne médiane de la tige, grâce à quoi les zones (26...29) de transition arrondies concaves sont formées chaque fois entre les surfaces des boules voisines.

2. Tige de prothèse selon la revendication 1, caractérisée en ce que les centres des boules voisines (23...25) se trouvent à l'extérieur des zones de pénétration respectives.

3. Tige de prothèse selon la revendication 1 ou 2, caractérisée en ce que les boules (23...25), de l'extrémité proximale à l'extrémité distale de la tige, ont des rayons allant en décroissant.

4. Tige de prothèse selon l'une des revendications 1 à 3, caractérisée en ce que les boules (23...25) sont formées à l'intérieur de l'espace creux (33) disponible dans l'os, avec des rayons aussi grands que possible.

5. Tige de prothèse selon l'une des revendications 1 à 4, caractérisée en ce que, dans la partie médiane de la longueur de la tige, une zone (2 4) est prévue avec une section transversale non circulaire.

FIG. 1

FIG. 2

20
21
30
26
22
23
27
24
28
25
29
31

FIG. 3

36
35

FIG. 4

FIG. 5

FIG. 6